# EUROPEAN PATENT APPLICATION

(11) **EP 3 705 155 A1**
(43) Date of publication of application: **09.09.2020**
(21) Application number: 18873082.4
(22) Date of filing: 01.11.2018
(51) Int. Cl.: A61M 37/00

(54) **DENTAL LOCAL ANESTHETIC MICRONEEDLE ARRAY**

(30) Priority: 02.11.2017 JP 2017213296
(71) Applicant: Cosmed Pharmaceutical Co., Ltd., Kyoto-shi, Kyoto 601-8014 (JP)
(72) Inventor: QUAN, Ying-shu, Kyoto-shi Kyoto 601-8014 (JP); KAMIYAMA, Fumio, Kyoto-shi Kyoto 601-8014 (JP)
(74) Representative: Ter Meer Steinmeister & Partner
(86) International application number: PCT/JP2018/040719
(87) International publication number: WO 2019/088227

(57) **Abstract**

The invention provides a microneedle array easily applied to an oral cavity and may exert an anesthetic effect according to an application site. Provided are an immediate-acting dental local anesthetic preparation including a microneedle array containing a local anesthetic, in which a needle part dissolves in a mucous membrane when being applied to an oral mucous membrane or gums, the microneedle array containing the local anesthetic, in which a height of a microneedle is 50 µm or more and 300 µm or less, a tip of the microneedle is a circle having a diameter of 1 µm or more and 50 µm or less or a plane having the same area, and a thickness of a substrate of the microneedle array is 5 µm or more and 100 µm or less, and a microneedle patch including the microneedle array and a support provided on a back surface of the microneedle array.

## Description

### TECHNICAL FIELD

The present invention relates to a technical field of a microneedle applied to dental (oral) local anesthesia.

### BACKGROUND ART

In dental treatment, local anesthesia is applied to an oral cavity to reduce pain, and an anesthetic is applied to an oral cavity (gingival) mucous membrane or the anesthetic is injected into gums.

Many commercially available dental local anesthetics are used. They are mainly local anesthetic-containing liquids, gels, jellies and the like; absorbent cotton and the like are immersed in the liquids to be applied to the oral cavity. The gels and jellies are applied directly to the oral cavity. In both cases, since absorption of the anesthetic from the mucous membrane is slow, it takes a long time for an effect to occur, a patient often lies down and waits for a long period time, transmucosal absorption fluctuates, so that quality of life often becomes impaired. There also is a disadvantage of a local surface anesthetic that the anesthetic flows from an application site and a wide part in the oral cavity is anesthetized. Anesthetic injection is painful at the time of injection of anesthetic and increases fear before treatment, which is one of reasons for avoiding dental treatment.

Microneedle preparations have high transdermal absorbability, and development of cosmetic products and pharmaceutical agents has been attempted. In general, an application site of the microneedle preparation is the skin epidermis, but for example, a microneedle patch for vaccination by intrabuccal administration is known (Patent Document 1). This microneedle patch is designed to penetrate an outer layer of the intrabuccal mucous membrane. In addition, a microneedle for transmitting dental substances such as dental local anesthetic is developed (Patent Documents 2 and 3). Patent Document 2 includes a microneedle array and a hollow spherical container containing a dental local anesthetic therein, and the anesthetic is locally delivered through an opening that penetrates the microneedle. Patent Document 3 discloses a microneedle provided with a base part that bends along a skin shape inside the oral cavity, a microneedle main body, and an active ingredient coating part coated on a surface of the needle main body.

### PRIOR ART DOCUMENTS

### PATENT DOCUMENTS

Patent Document 1: JP 2015-515474 A
Patent Document 2: JP 2017-507734 A
Patent Document 3: JP 2017-061447 A

### SUMMARY OF THE INVENTION

### PROBLEM TO BE SOLVED BY THE INVENTION

Although an attempt has been made to apply the microneedle technique to dental local anesthesia, a device as a microneedle device is complicated, so that there is a demand for a microneedle preparation that is more simple and can provide an anesthetic effect. An object of the present invention is to provide a microneedle array which is easily applied to the oral cavity and can exert an anesthetic effect according to an application site.

### MEANS FOR SOLVING THE PROBLEM

If rapid surface anesthesia may be performed to the depth of 1 to 2 mm in the skin before the injection of anesthetic, the patient's quality of life may be significantly improved. As a result of intensive studies in view of the specialty of the oral cavity tissue and the dental field, the present inventors have achieved a microneedle array suitable for dental local anesthesia by specifying the shape and material of the microneedle array.

The present invention is as follows.
[1] An immediate-acting dental local anesthetic preparation including a microneedle array containing a local anesthetic, in which a needle part dissolves in a mucous membrane when being applied to an oral mucous membrane or gums.
[2] The dental local anesthetic preparation according to [1], in which a back surface of the microneedle array is lined with a hydrophobic or non-dissolving film.
[3] The dental local anesthetic preparation according to [1] or [2], in which the microneedle array has a water-soluble polymer as a base, and includes a flexible substrate having a thickness of 100 µm or less.
[4] The dental local anesthetic preparation according to [3], in which the water-soluble polymer is one or two or more types selected from the group consisting of hyaluronic acid and its derivative, collagen, proteoglycan, hydroxypropyl cellulose, chondroitin sulfate, carboxymethyl cellulose, polyvinyl pyrrolidone, polyethylene glycol, and dextran.
[5] The dental local anesthetic preparation according to [3] or [4], in which the base of the microneedle array contains 2% by mass or more of a water-soluble low-molecular compound in addition to the water-soluble polymer.
[6] The dental local anesthetic preparation according to any one of [1] to [5], in which the local anesthetic is selected from the group consisting of procaine, tetracaine, lidocaine, dibucaine, bupivacaine and salts thereof.
[7] The dental local anesthetic preparation according to any one of [1] to [5], in which the local anesthetic is ethyl aminobenzoate.
[8] The dental local anesthetic preparation according to any one of [1] to [5], in which the local anesthetic is a mixture of one or more selected from the group consisting of procaine, tetracaine, lidocaine, dibucaine, bupivacaine and salts thereof, and ethyl aminobenzoate.
[9] The dental local anesthetic preparation according to any one of [1] to [8], in which a concentration of the local anesthetic in the base is 1% by mass or more and 80% by mass or less.
[10] The dental local anesthetic preparation according to any one of [1] to [6], [8], and [9], in which the local anesthetic is lidocaine or salt thereof.
[11] A microneedle array having a water-soluble polymer as a base and containing a local anesthetic, in which a height of a microneedle is 50 µm or more and 300 µm or less, a tip of the microneedle is a circle having a diameter of 1 µm or more and 50 µm or less or a plane having the same area, and a thickness of a substrate of the microneedle array is 5 µm or more and 100 µm or less.
[12] The microneedle array according to [11], in which the water-soluble polymer is one or two or more types selected from the group consisting of hyaluronic acid and its derivative, collagen, proteoglycan, hydroxypropyl cellulose, chondroitin sulfate, carboxymethyl cellulose, polyvinyl pyrrolidone, polyethylene glycol, and dextran.
[13] The microneedle array according to [11] or [12], in which the base contains 2% by mass or more of a water-soluble low-molecular compound in addition to the water-soluble polymer.
[14] The microneedle array according to any one of [11] to [13], in which the local anesthetic is selected from the group consisting of procaine, tetracaine, lidocaine, dibucaine, bupivacaine and salts thereof.
[15] The microneedle array according to any one of [11] to [13], in which the local anesthetic is ethyl aminobenzoate.
[16] The microneedle array according to any one of [11] to [13], in which the local anesthetic is a mixture of one or more selected from the group consisting of procaine, tetracaine, lidocaine, dibucaine, bupivacaine and salts thereof, and ethyl aminobenzoate.
[17] The microneedle array according to any one of [11] to [16], in which a concentration of the local anesthetic in the base is 1% by mass or more and 80% by mass or less.
[18] The microneedle array according to any one of [11] to [14], [16], and [17], in which the local anesthetic is lidocaine or salt thereof.
[19] A microneedle patch including: the microneedle array according to any one of [11] to [18]; and a support provided on a back surface of the microneedle array.
[20] The microneedle patch according to [19], in which the support has intraoral adhesiveness.
[21] The microneedle patch according to [20], in which the support is coated with an adhesive substance.
[22] The microneedle patch according to [20], in which the support is water soluble.
[23] The microneedle patch according to any one of [19] to [22], in which the support has a film shape and includes an absent part not containing a film in a part.
[24] The microneedle patch according to any one of [19] to [22], in which the support is sterilized paper and forms an outer frame enclosing the microneedle array.

### EFFECT OF THE INVENTION

The microneedle array of the present invention is easily manufactured because the substrate and the microneedles are integrally formed using the water-soluble polymer as the base, and by adjusting the amount of local anesthetic contained therein and the size of the microneedle array, it is possible to achieve an anesthetic effect corresponding to the purpose in a short time.

Since the microneedle array and the microneedle patch of the present invention use the water-soluble polymer as the base, they easily adhere following bending of the oral mucous membrane or the gums in a high-humidity environment, and are suitable for local administration in the oral cavity.

The microneedle array and the microneedle patch of the present invention can be used as a dental local anesthetic preparation, and also as a pre-anesthetic for reducing pain at an administration site before administering a dental local anesthetic injection solution.

### BRIEF DESCRIPTION OF THE DRAWINGS

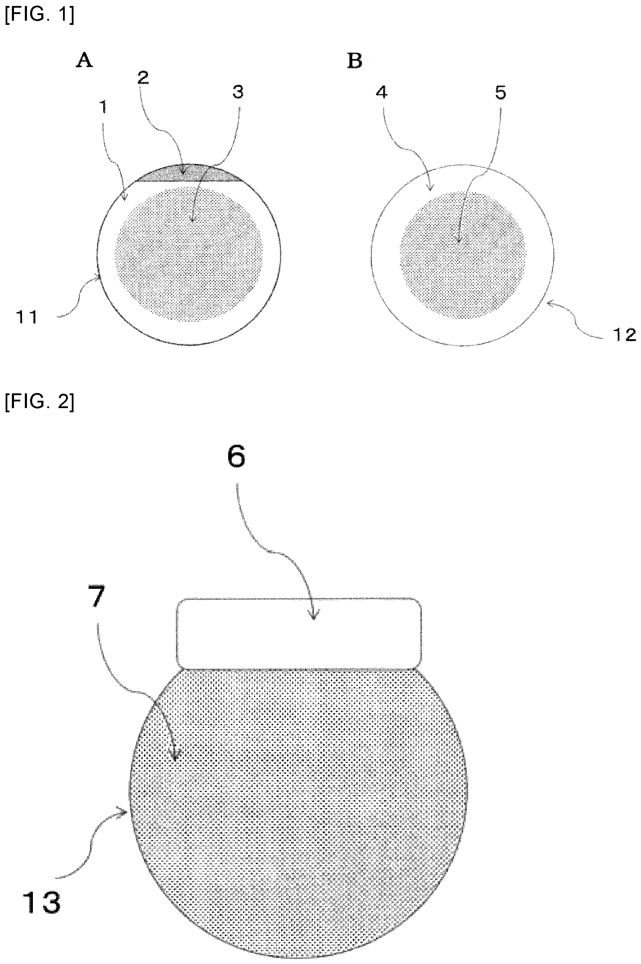

FIG. 1 is a schematic diagram of a microneedle patch of the present invention. In FIG. 1A, a polyethylene adhesive film 1 is used as a support, but there is no polyethylene adhesive film at the center of a back surface of a microneedle part 3. In FIG. 1B, sterilized paper 4 is used as a support, and the sterilized paper is not present on a back surface of the microneedle part 5, and this forms an outer frame of the microneedle patch.
FIG. 2 is a schematic diagram of a microneedle patch having no support at the center of a back surface of a microneedle part 7 with a tab for holding by hand at a part of an end.

### MODE FOR CARRYING OUT THE INVENTION

A microneedle array of the present invention is suitable for local anesthesia, especially dental local anesthesia. The microneedle array of the present invention is formed of a substrate and a plurality of microneedles on the substrate integrally formed of the same water-soluble polymer as a base.

### (Base of microneedle array)

The base of the microneedle array is the water-soluble polymer. When the microneedle array containing a local anesthetic uniformly is prepared using such a material by a conventional method, the local anesthetic is contained not only in a microneedle part but also in a substrate. When this microneedle array is applied to an oral cavity (oral mucous membrane, gums or the like), the microneedle part may reach inside the mucous membrane or gums, so that the microneedle part dissolves in the mucous membrane and promotes delivery of local anesthetic contained therein to a target site. The substrate of the microneedle array also adheres following bending of the oral mucous membrane or gums in a high-humidity environment in the oral cavity, the water-soluble polymer of the substrate dissolves, and the local anesthetic present there is also delivered to the target site.

Examples of the water-soluble polymer include hyaluronic acid and its derivative (for example, sodium salt, polyethylene oxide grafted hyaluronic acid), collagen, proteoglycan, hydroxypropyl cellulose, chondroitin sulfate, carboxymethyl cellulose, polyvinyl pyrrolidone, polyethylene glycol or dextran, and one or two or more selected from them may be mixed and used. Especially, hyaluronic acid or its derivative is preferable.

Hyaluronic acid is a type of glycosaminoglycan (mucopolysaccharide) and has a structure in which disaccharide units of N-acetylglucosamine and glucuronic acid are linked. Examples of hyaluronic acid include living organism-derived hyaluronic acid isolated from cockscombs, umbilical cords and the like, culture-derived hyaluronic acid mass-produced by lactic acid bacteria, streptococci and the like, for example. From living organism-derived hyaluronic acid, collagen of the living organism from which this is derived cannot be completely removed, and remaining collagen might have an adverse effect, so that culture-derived hyaluronic acid that does not contain collagen is preferred. Therefore, hyaluronic acid preferably contains 50% by mass or more of culture-derived hyaluronic acid.

When preparing the microneedle array using water-soluble polymer substances selected from hyaluronic acid or its derivative as ingredients, the microneedle array formed from the polymer substances tends to be harder and easily stick in an application site as a weight-average molecular weight thereof decreases, and tends to be softer and easily applied to bending of the gums and the like as the weight-average molecular weight thereof increases and mechanical strength improves to increase stiffness. For the purpose of the present invention, the weight-average molecular weight is preferably 5,000 to 2,000,000.

When applying the microneedle array in the oral cavity, the microneedle array may be formed of a mixture of high-molecular weight polymer substances having the weight-average molecular weight of 100,000 or more and low-molecular weight polymer substances having the weight-average molecular weight of 50,000 or less in order to make the same appropriately hard to be hardly broken and make the local anesthetic to be easily penetrated. The weight-average molecular weight of the high-molecular weight polymer substances may be 50,000 or more, and preferably 2,000,000 or less. The weight-average molecular weight of the low-molecular weight polymer substances may be 50,000 or less, and preferably 1,000 or more. In the present invention, the weight-average molecular weight is a value measured by gel permeation chromatography (GPC).

A ratio when the high-molecular weight polymer substances and the low-molecular weight polymer substances are mixed varies depending on the type and weight-average molecular weight of each polymer substance, so that this may be appropriately determined so as to obtain preferable mechanical strength and hardness; however, in general, this is preferably 1% by mass or more of the high-molecular weight polymer substances and 99% by mass or less of the low-molecular weight polymer substances.

In order to exert an anesthetic effect quickly, a soluble agent may be added to the polymer substance. Examples of the soluble agent include monosaccharides such as trehalose and glucose, disaccharides, polyhydric alcohols such as glycerin, propylene glycol (PG), butylene glycol (BG), and polyethylene glycol (PEG) and the like. An additive amount of the soluble agent is desirably 1% by mass or more and 50% by mass or less as a concentration in the base.

In order to prevent drug crystallization, polyvinylpyrrolidone (PVP) or dextran may be added.

The base of the microneedle array may contain a water-soluble low-molecular compound in addition to the water-soluble polymer. Examples of the water-soluble low-molecular compound include monosaccharides, disaccharides, and polyhydric alcohols used as the soluble agent described above, the compounds having a molecular weight of 500 or less. Examples of monosaccharides include glucose, fructose and the like, and examples of disaccharides include sucrose, lactose, trehalose, maltose and the like. Examples of polyhydric alcohols include glycerin, propylene glycol (PG), butylene glycol (BG), polyethylene glycol (PEG) 200, PEG 400 and the like.

An additive amount of the water-soluble low-molecular compound is 2% by mass or more and 50% by mass or less, preferably 2% by mass or more and 35% by mass or less, more preferably 2% by mass or more and 30% by mass or less as a concentration in the base.

### (Shape of microneedle array)

A height of the microneedle is desirably 50 µm or more and 300 µm or less, and more preferably 100 µm or more and 250 µm or less. When this is 50 µm or less, it is disadvantageous for delivery of the local anesthetic. When this exceeds 300 µm, application might be accompanied by pain and bleeding.

A tip of the microneedle is desirably a circle having a diameter of 1 µm or more or a plane having the same area. The tip of the microneedle is desirably a circle having a diameter of 50 µm or less or a plane having the same area. Within this range, it is advantageous for the delivery of the local anesthetic. Examples of the needle shape include a bar shape, a truncated cone shape, or a conide, and the truncated cone shape or the conide shape is desirable.

The microneedle array preferably includes a flexible substrate. A thickness of the substrate of the microneedle array is desirably 5 µm or more and 100 µm or less, and more preferably 10 µm or more and 50 µm or less.

The shape of the substrate of the microneedle array may be appropriately set according to the application site, and examples thereof include a circle, an ellipse, a triangle, a quadrangle, a polygon and the like. A size of the shape is 2 mm or more and 100 mm or less in general, and preferably 5 mm or more and 50 mm or less when represented by a diameter (major axis) or a length of one side (long side). In addition, when a size of the microneedle array is represented in terms of area, this is usually 5 mm² or more and 1000 mm² or less, preferably 10 mm² or more and 500 mm² or less.

### (Local anesthetic)

An active ingredient contained in the microneedle array of the present invention is the local anesthetic. Examples of the local anesthetic include procaine, tetracaine, lidocaine, dibucaine, bupivacaine, or salts thereof. Alternatively, the local anesthetic may also be ethyl aminobenzoate (benzocaine) .

In the present invention, two or more types of these local anesthetics may be mixed to be used. A preferred combination is a combination (mixture) of one or more selected from the group consisting of procaine, tetracaine, lidocaine, dibucaine, bupivacaine and salts thereof and ethyl aminobenzoate.

When the local anesthetic is used alone, lidocaine or a salt thereof is preferred, and lidocaine hydrochloride salt is preferred as the salt of lidocaine.

In addition to the local anesthetic, an additive usually contained as a pharmaceutical agent may also be contained. A concentration of the additive contained in the microneedle array of the present invention may be set in an appropriate range according to the type and purpose of the additive.

A concentration of the local anesthetic in the base is 1% by mass or more and 80% by mass or less, and more preferably 10% by mass or more and 70% by mass or less. Herein, the concentration of the local anesthetic in the base is mass in the total weight of the microneedle array (drag content in solid mass of the microneedle array obtained by dissolving the microneedle array in an appropriate solvent such as water and quantitively analyzing content of the local anesthetic).

A method of manufacturing the microneedle array of the present invention is not especially limited, and this may be manufactured by any conventionally known method; for example, there is a method of casting an aqueous solution containing the above-described water-soluble polymer and local anesthetic, and other ingredients as needed in a mold in which a shape of the microneedle is bored and peeling the same after drying. A peeled microneedle array sheet is used after being cut according to a shape of the application site in the oral cavity.

The microneedle array of the present invention may be used alone as a dental local anesthetic preparation. Alternatively, for the convenience of intraoral application, this may be made the following microneedle patch.

### (Microneedle patch)

The microneedle patch of the present invention is formed of the microneedle array and a support provided on a back surface of the microneedle array. Herein, the back surface of the microneedle array is a substrate on a side opposite to a surface from which the microneedles protrude. Although the support is not indispensable, it is easy to handle if there is the support, and it is possible to prevent slip from an application site or movement to the inside of the lips. The microneedle patch obtained by lining the back surface of the microneedle array with a hydrophobic or non-dissolving film as the support is an embodiment of the dental local anesthetic preparation. The dental local anesthetic preparation is an immediate-acting dental local anesthetic preparation having an immediate effect.

Preparation formulation of the present invention may have various aspects. They are described sequentially.
1. The microneedle patch obtained by lining the back surface of the microneedle array manufactured by the method of manufacturing the microneedle array and dried with a polymer film as the support. There are various manufacturing methods. For example, the microneedle array is dried, and before peeling the same from the mold, a polymer dissolved in water or a low-boiling point organic solvent is laminated on the back surface thereof by application, spraying or the like, and dried. Herein, the polymer is a water-soluble polymer such as polyvinyl alcohol, high-molecular weight polyvinyl pyrrolidone, hydroxypropyl cellulose, or polyacrylic acid, the polymer which does not dissolve instantaneously in the oral cavity. More specifically, it is necessary for the microneedle substrate not to be dissolved or to be deformed at least for 30 minutes after application because of the lining of the polymer film as the support. The support may be an organic solvent-soluble polymer such as polyvinyl acetate, polyvinyl chloride, or nylon, or those made flexible by a plasticizer. They are preferred specific examples of the hydrophobic or non-dissolving film.
2. The microneedle patch obtained by lining the back surface of the microneedle array manufactured by the method of manufacturing the microneedle array and dried with a polymer film as the support. This preparation is such that the polymer film is integrated with the back surface of the microneedle array with a bonding agent or an adhesive. Sizes of the microneedle array and the polymer film may be similar to each other, or the polymer film may be larger and a film surface thereof may be treated to have an intraoral bonding property. The polymer film may be water-permeable such as a porous or woven fabric. Typically, a plastic sheet or a film of polyethylene, polypropylene, polyethylene terephthalate, ethylene vinyl acetate copolymer (EVA) and the like; a paper sheet such as sterilized paper, cellophane, non-woven fabric, and woven fabric; a silicon resin thin film by spraying or application; a fluorine oil thin film by spraying or application and the like are included.

The support may be of the same type and same size as those of the microneedle array, but this is preferably larger than the microneedle array in order to reinforce adhesive force of the microneedle array in the oral cavity from the back surface. The support may be set to have the size and shape easy to handle depending on the application site; for example, it is appropriate to make the same larger by approximately 3 to 20 mm from an outer edge of the microneedle array. A thickness of the support may be equivalent to or thicker or thinner than the thickness of the microneedle array substrate; this may be appropriately set to the thickness capable of supporting a flexible and thin microneedle array and easy to handle. A shape like a tab for holding by hand may be present at an end of the microneedle array (FIG. 2, polyethylene adhesive film 6). A part or an entire surface of the support may be colored; after a doctor finishes anesthetizing, it is easy to remove the same property if there is a colored mark.

The support desirably has intraoral adhesiveness in order to reinforce the adhesive force of the microneedle array in the oral cavity from the back surface.

As one aspect for securing the intraoral adhesiveness of the support, there is a support in which the support is coated with an adhesive substance, that is, a support coated with an adhesive. Herein, as the adhesive substance, the adhesive normally used for a patch preparation is mentioned; for example, a grade with a wet surface bonding property of an acrylic type, a silicone type, and a rubber type adhesive is preferable.

Another aspect for securing the intraoral adhesiveness of the support is that the support is water-soluble. The one using a low-molecule weight water-soluble film of polyvinyl pyrrolidone (PVP), carboxymethyl cellulose (CMC), polyvinyl alcohol (PVA) and the like having self-adhesiveness with moisture in the oral cavity is also preferable. In this case, it is desirable to further laminate a water-insoluble polymer film on a surface facing the oral cavity surface opposite to the water-soluble support so as to prevent bonding to the oral cavity surface opposite to the oral cavity surface of application.

The film laminated on the back surface is effective because the back surface of the microneedle array tends to adhere to the oral mucous membrane on the opposite side of the mucous membrane of the application site without same. However, this is not an essential requirement of the present invention, and the essential requirement of the present invention is drug delivery to the deep mucous membrane by the microneedle. In a case where the microneedle base is water-soluble but its water dissolution rate is low, drug dissolution at the microneedle part is much faster than that of the back surface, so that the purpose may be achieved even without a lining agent. That is, the microneedle array of the present invention itself is provided as the dental local anesthetic preparation.

In a case of a film-shaped support, a part thereof may be an absent part not containing the film. For example, as illustrated in FIG. 1A, the absent part may be provided at the center of the film-shaped support, and in this case, the back surface of the microneedle part is not covered with the film. The absent part is not limited to the central portion, and it is sufficient to secure a portion not including the film to the extent that needle insertion is not prevented in a case where an injection needle is inserted into a site to which the microneedle patch of the present invention is applied. By not providing the support at the center of the microneedle array, it is possible to inject directly from the back surface without removing the microneedle patch in the case of pre-anesthesia. In addition, it may be tested from the back surface whether the anesthetic effect is sufficient.

Similarly, in a case where the support is the sterilized paper, the support may form an outer frame that encloses the microneedle array. For example, as illustrated in FIG. 1B, a hole is provided at the center of the sterilized paper, the back surface of the microneedle part is not covered with the sterilized paper, and the sterilized paper forms the outer frame of the microneedle array. The outer frame may be provided to such a degree that the sterilized paper is prevented from covering the entire back surface of the substrate of the microneedle array to prevent penetration of the needle in a case where the injection needle sticks in the site to which the microneedle patch of the present invention is applied.

The microneedle patch of the present invention may be manufactured by covering the back surface of the microneedle array with the support.

After applying the microneedle array and the microneedle patch of the present invention to the oral mucous membrane or gums, when the back surface of the microneedle part is pressed, the local anesthetic is administered. Since the microneedle array and the microneedle patch of the present invention use the water-soluble polymer as the base, they may be quickly dissolved under a high humidity environment and the anesthetic may be efficiently delivered into the oral mucous membrane or gums, so that the effect of local anesthesia may be exerted in a short time (within 1 to 10 minutes). Evaluation of the preparation may be confirmed by a test of applying the same to the gums of a volunteer, peeling the same after 5 to 10 minutes, and sticking a toothpick or injection needle in the application site to check whether the volunteer feels pain. At that time, by applying a rubber ring to a position 1 mm from a tip of the toothpick or the injection needle as a stopper, thereby preventing the toothpick or the injection needle from entering deeper than 1 mm in the gum even when they are strongly pushed.

By appropriately setting an amount of the local anesthetic contained in the microneedle array per unit area and the size of the microneedle array, this may be used as the dental local anesthetic preparation. This may also be used as a pre-anesthetic for reducing pain at an administration site before administering a dental local anesthetic injection solution. In this case, after applying the microneedle array and the microneedle patch of the present invention to the oral mucous membrane or gums, the dental local anesthetic injection may be subsequently applied to the application site.

### EXAMPLES

Hereinafter, the present invention is described with reference to examples; however, the present invention is not limited to the examples.

### (Example 1)

### (Manufacture of microneedle patch containing local anesthetic)

50 parts by mass of lidocaine hydrochloride (purchased from WAKENYAKU CO., LTD.) and 50 parts by mass of sodium hyaluronate (FCH-SU, Kikkoman Corporation) were measured, and water was added to prepare a solution having a solid content of 10% by mass. The aqueous solution was casted to a mold having a needle length of 200 µm, dried at room temperature for 24 hours, and punched to produce a microneedle array. Thereafter, a perforated polyethylene (PE) adhesive film was bonded to a back surface of the array.

### (Example 2)

Ethyl aminobenzoate (purchased from WAKENYAKU CO., LTD.) was dissolved with ethanol and mixed in a mixed aqueous solution of 10% by mass of hydroxypropylcellulose and PEG1000 (Nippon Bulk Yakuhin Co., Ltd.) (HPCL: PEG1000 = 10: 0.5), and the mixture was filled in a mold and dried. Content of ethyl aminobenzoate in the microneedle patch was 20% by mass. Before this is peeled off from the mold, a 10% by mass of ethyl acetate solution of polyvinyl acetate was applied thereto and dried at 60°C for 20 minutes, then punched into an oval shape with a short axis of 1 cm and a long axis of 2 cm to obtain a microneedle preparation with a support (support thickness = 40 µm, microneedle substrate thickness = 50 µm).

This preparation was applied to the gums of five volunteers and peeled off after 5 minutes, then it was tested whether or not pain was felt while sticking a toothpick in an application site. All did not feel pain and an anesthetic effect was confirmed.

### (Example 3)

The following drug-containing microneedle patch was prepared in a manner similar to that in the Example 2.
Benzocaine (ethyl aminobenzoate) 25% by mass
Tetracaine hydrochloride 1% by mass
Dibucaine hydrochloride 1% by mass
Homosulfamine 2% by mass

Before this is peeled off from the mold, a 30% by mass aqueous solution of polyvinyl alcohol was applied thereto and dried at 60°C for 20 minutes, then punched into an oval shape with a short axis of 1 cm and a long axis of 2 cm to obtain a microneedle preparation with a support (support thickness = 30 µm, microneedle substrate thickness = 50 µm).

This preparation was applied to the gums of five volunteers and peeled off after 5 minutes, then it was tested whether or not pain was felt while sticking a toothpick in an application site. All did not feel pain and an anesthetic effect was confirmed.

### (Examples 4 to 9, Comparative Examples 1 and 2)

Microneedle preparations containing bases and anesthetics listed in Table 1 were manufactured according to the method described in the Example 2 (Examples 4 to 9). However, the microneedle preparation of the Example 6 had no support (thickness of the substrate was 100 µm). The microneedle preparations of Examples 4, 5, and 7 to 9 were each a microneedle preparation with a support using lining agents listed in Table 1 (microneedle substrate thickness = 40 to 50 µm, support thickness = 40 to 60 µm).

As comparative examples, a needleless sheet preparation of microneedles (Comparative Example 1) and gel ointment preparation (Comparative Example 2) were manufactured based on compositions in Table 1.

**[Table 1]**

| Example Comparative Example | Anesthetic % in base | Base | Lining agent | Needle strength N | Anesthetic effect |
|---|---|---|---|---|---|
| Example 4 | Ethyl aminobenzoate 10% Diethylaminoethyl p-butylaminobenzoate hydrochloride 5% | Hyaluronic acid 70% Glucose 30% | Polyethylene adhesive film | 138 | Excellent in 5 minutes |
| Example 5 | Tetracaine hydrochloride 20% | Hydroxypropyl cellulose 80% Trehalose 20% | Polyvinyl acetate | 152 | Excellent in 5 minutes |
| Example 6 | Lidocaine 30% | Hyaluronic acid | None | 166 | Excellent in 5 minutes |
| Example 7 | Ethyl aminobenzoate 20% Lidocaine hydrochloride 15% | Hyaluronic acid 95% Glycerin 5% | Polyvinyl alcohol | 134 | Excellent in 5 minutes |
| Example 8 | Lidocaine hydrochloride 2% | Polyvinylpyrrolidone (PVP) 70% Dextran 30% | Acrylic adhesive/ 25 µm PU tape | 155 | Excellent in 10 minutes |
| Example 9 | Lidocaine hydrochloride 4% | Polyvinyl alcohol (PVA) 70% Maltose 30% | Acrylic adhesive/ 16 µm PET tape | 146 | Excellent in 5 minutes |
| Comparative Example 1 Needleless sheet | Lidocaine 30% | Hyaluronic acid | None | - | Poor in 10 minutes |
| Comparative Example 2 Gel ointment | Ethyl aminobenzoate 20% | Saccharin sodium hydrate Macrogol Fragrance Water | None | - | Poor in 10 minutes |

| | | | | | |
|---|---|---|---|---|---|
| % represents % by mass | | | | | |

### (Needle strength test)

The microneedle arrays molded in the Examples 4 to 9 were subjected to a compression test using a small desktop testing machine EZ Test EZSX (manufactured by Shimadzu Corporation) to measure the mechanical strength of the needles. The microneedle array was molded to have a diameter of 1 cm, fixed between two stainless steel plates, and compressed at a speed of 1 mm/min to obtain a stress/strain curve.

From the stress/strain curve, an elastic modulus was obtained as a criterion for evaluating the mechanical strength of the needle to be compared. The elastic modulus was calculated from a linear gradient at the strain of 0.1 to 0.2 mm, which is an initial steady state in the stress/strain curve in which stress is plotted along the ordinate and strain is plotted along the abscissa. Results are illustrated in Table 1.

### (Anesthetic effect)

The preparations manufactured in the Examples 4 to 9 and the Comparative Examples 1 and 2 were applied to the gums of five volunteers and peeled off after 5 to 10 minutes, then it was tested whether or not pain was felt while sticking a toothpick in an application site. The criteria for anesthetic evaluation were as follows. Results are illustrated in Table 1.
No one feels pain: excellent effect
Three to four people do not feel pain: effective
Zero to two people do not feel pain: poor effect

Each of the microneedle preparations of the Examples 4 to 9 was able to exert an anesthetic effect on all volunteers within 10 minutes. It was difficult for the sheet preparation and the gel ointment to exert the anesthetic effect within 10 minutes.

### DESCRIPTION OF REFERENCE SYMBOLS

- 1: Polyethylene adhesive film
- 2: Adhesive-free polyethylene film
- 3: Microneedle part
- 4: Sterilized paper
- 5: Microneedle part
- 6: Polyethylene adhesive film
- 7: Microneedle part
- 11: Microneedle patch
- 12: Microneedle patch
- 13: Microneedle patch

## Claims

1. An immediate-acting dental local anesthetic preparation comprising a microneedle array containing a local anesthetic, wherein a needle part dissolves in a mucous membrane when being applied to an oral mucous membrane or gums.

2. The dental local anesthetic preparation according to claim 1, wherein a back surface of the microneedle array is lined with a hydrophobic or non-dissolving film.

3. The dental local anesthetic preparation according to claim 1 or 2, wherein the microneedle array has a water-soluble polymer as a base, and a flexible substrate having a thickness of 100 µm or less.

4. The dental local anesthetic preparation according to claim 3, wherein the water-soluble polymer is one or two or more types selected from the group consisting of hyaluronic acid and its derivative, collagen, proteoglycan, hydroxypropyl cellulose, chondroitin sulfate, carboxymethyl cellulose, polyvinyl pyrrolidone, polyethylene glycol, and dextran.

5. The dental local anesthetic preparation according to claim 3 or 4, wherein the base of the microneedle array contains 2% by mass or more of a water-soluble low-molecular compound in addition to the water-soluble polymer.

6. The dental local anesthetic preparation according to any one of claims 1 to 5, wherein the local anesthetic is selected from the group consisting of procaine, tetracaine, lidocaine, dibucaine, bupivacaine and salts thereof.

7. The dental local anesthetic preparation according to any one of claims 1 to 5, wherein the local anesthetic is ethyl aminobenzoate.

8. The dental local anesthetic preparation according to any one of claims 1 to 5, wherein the local anesthetic is a mixture of one or more selected from the group consisting of procaine, tetracaine, lidocaine, dibucaine, bupivacaine and salts thereof, and ethyl aminobenzoate.

9. The dental local anesthetic preparation according to any one of claims 1 to 8, wherein a concentration of the local anesthetic in the base is 1% by mass or more and 80% by mass or less.

10. The dental local anesthetic preparation according to any one of claims 1 to 6, 8, and 9, wherein the local anesthetic is lidocaine or salt thereof.

11. A microneedle array having a water-soluble polymer as a base and containing a local anesthetic, wherein a height of a microneedle is 50 µm or more and 300 µm or less, a tip of the microneedle is a circle having a diameter of 1 µm or more and 50 µm or less or a plane having the same area, and a thickness of a substrate of the microneedle array is 5 µm or more and 100 µm or less.

12. The microneedle array according to claim 11, wherein the water-soluble polymer is one or two or more types selected from the group consisting of hyaluronic acid and its derivative, collagen, proteoglycan, hydroxypropyl cellulose, chondroitin sulfate, carboxymethyl cellulose, polyvinyl pyrrolidone, polyethylene glycol, and dextran.

13. The microneedle array according to claim 11 or 12, wherein the base contains 2% by mass or more of a water-soluble low-molecular compound in addition to the water-soluble polymer.

14. The microneedle array according to any one of claims 11 to 13, wherein the local anesthetic is selected from the group consisting of procaine, tetracaine, lidocaine, dibucaine, bupivacaine and salts thereof.

15. The microneedle array according to any one of claims 11 to 13, wherein the local anesthetic is ethyl aminobenzoate.

16. The microneedle array according to any one of claims 11 to 13, wherein the local anesthetic is a mixture of one or more selected from the group consisting of procaine, tetracaine, lidocaine, dibucaine, bupivacaine and salts thereof, and ethyl aminobenzoate.

17. The microneedle array according to any one of claims 11 to 16, wherein a concentration of the local anesthetic in the base is 1% by mass or more and 80% by mass or less.

18. The microneedle array according to any one of claims 11 to 14, 16, and 17, wherein the local anesthetic is lidocaine or salt thereof.

19. A microneedle patch comprising:
the microneedle array according to any one of claims 11 to 18; and
a support provided on a back surface of the microneedle array.

20. The microneedle patch according to claim 19, wherein the support has intraoral adhesiveness.

21. The microneedle patch according to claim 20, wherein the support is coated with an adhesive substance.

22. The microneedle patch according to claim 20, wherein the support is water soluble.

23. The microneedle patch according to any one of claims 19 to 22, wherein the support has a film shape and includes an absent part not containing a film in a part.

24. The microneedle patch according to any one of claims 19 to 22, wherein the support is sterilized paper and forms an outer frame enclosing the microneedle array.
